# EUROPEAN PATENT SPECIFICATION

(11) **EP 2 756 296 B1**
(45) Date of publication and mention of the grant of the patent: **15.11.2017**
(21) Application number: 12783251.7
(22) Date of filing: 12.09.2012
(51) Int. Cl.: G01N 33/49, G01N 21/76, G01N 15/14, G01N 15/12, G06F 19/00

(54) **DIAGNOSTIC APPARATUS**
DIAGNOSEGERÄT
APPAREIL DE DIAGNOSTIC

(30) Priority: 13.09.2011 GB 201115843
(43) Date of publication of application: 23.07.2014
(73) Proprietor: Orreco Limited, Sligo (IE)
(72) Inventor: MOORE, Brian, Sigo (IE)
(74) Representative: Beresford, Keith Denis Lewis
(86) International application number: PCT/GB2012/000712
(87) International publication number: WO 2013/038127

(56) References cited:
- WO-A1-01/33215
- WO-A1-2011/114145
- YAMADA M ET AL: "Effects of exhaustive exercise on human neutrophils in athletes", LUMINESCENCE, vol. 15, no. 1, 1 January 2000 (2000-01-01), pages 15-20, XP055051575, ISSN: 1522-7235
- Lee R: "Science of sailing", English Institute of Sports , 7 October 2008 (2008-10-07), XP055051667, Retrieved from the Internet: URL:http://www.eis2win.co.uk/pages/news_sc ienceofsailing.aspx [retrieved on 2013-01-30]
- QUINDRY J C ET AL: "The effects of acute exercise on neutrophils and plasma oxidative stress", MEDICINE & SCIENCE IN SPORTS & EXERCISE, vol. 35, no. 7, 1 July 2003 (2003-07-01), pages 1139-1145, XP055051796, ISSN: 0195-9131, DOI: 10.1249/01.MSS.0000074568.82597.0B
- KHAN J Y ET AL: "Wireless body area network (WBAN) design techniques and performance evaluation", JOURNAL OF MEDICAL SYSTEMS, vol. 36, no. 3, 16 October 2010 (2010-10-16), pages 1441-1457, XP035051450, ISSN: 1573-689X, DOI: 10.1007/S10916-010-9605-X
- KIKUCHI T ET AL: "Measurement of chemiluminescence from neutrophils in a 96-well microplate using Lumi Box U-800 II", JOURNAL OF BIOLUMINESCENCE AND CHEMILUMINESCENCE, vol. 12, 1 January 1997 (1997-01-01), pages 149-153, XP055051599, ISSN: 0884-3996

## Description

The present invention relates to diagnostic apparatus and techniques for blood analysis. More specifically, the present invention provides apparatus and methods which use measured blood parameters to determine a physical condition of a subject. From a subject's blood sample, the apparatus and techniques invention can provide an indication of the subject's "readiness to perform" so that a training regime may be adjusted to bring or return the subject to peak condition. The apparatus and techniques of the present invention can also provide an indication of the presence of an infection, and can do so before the subject exhibits external symptoms of the infection. In the present specification, references to "subject" should be interpreted as including references to either a human or an animal. In humans, the apparatus and techniques may be used in relation to competition athletes for monitoring their readiness to compete at their optimum level, or as a diagnostic tool for monitoring physical condition and/or stress in general subjects. In relation to animals, the techniques may be used for monitoring condition and/or stress in either agricultural animals or competition animals such as horses, camels, greyhounds or other animals used in racing or other competitions.

It has been observed in athletes that an accumulation of training stress, with insufficient rest and recovery periods, can result in a deterioration in performance. Continuing stress may also cause deterioration in performance in a subject in his workplace environment. In the case of athletes, in the short term, insufficient rest may result in the athlete becoming fatigued, and then "overreached", but in such cases a relaxation of the training regime is usually followed in a few days by a full recovery and possibly an improvement in performance. If the training is continued with insufficient rest for an extended period, the athlete may become "overtrained". In an overtrained athlete, the deterioration of performance and performance capacity may be accompanied by physiological and psychological symptoms, such as disturbed sleep patterns or changed heart rates, and an overtrained athlete may take weeks or months to recover from this state even if the training regime is relaxed. The training regime may be relaxed, for example, by the athlete training at lower intensity and/or for shorter periods.

Similar deterioration effects are seen for example in workplace performance in overstressed individuals, and depending on the period for which the overstress continues, recovery can be prolonged.

Detection of overreaching and overtraining has hitherto depended on monitoring the performance of the athlete and inferring fatigue, overreaching or overtraining on the basis of a deterioration in the athlete's performance. Using this technique, however, it is only possible to detect when the athlete has already passed his peak condition.

LUMINESCENCE, vol. 15, no. 1, 1st January 2000, pages 15-20, XP055051575, ISSN 1522-7235; YAMADA M et al: "Effects of exhaustive exercise on human neutrophils in athletes" describes the testing of athletes pre- and post-exercise to investigate the effects of acute exercise on the capacity of neutrophils to produce reactive oxygen species (ROS). The test protocol involves the use of chemiluminescence and the counting of leucocvtes using a a Lumi Box H-1000 (Microtec) luminometer and a Micro Diff II (Beckmann Coulter) cytometer.

MEDICINE & SCIENCE IN SPORTS & EXERCISE, vol. 35, no. 7, 1st July 2003, pages 1139-1145, XP055051796, ISSN 0195-9131;QUINDRY J C et al: "The effects of acute exercise on neutrophils and plasma oxidative stress" describes measuring, immediately after exercise, the effects of an acute exercise episode causing significant inflammation and disturbance to both white blood cell count and function. The apparatus for carrying out the technique comprises an American Optical Corp hemocytometer and Galaxy Floustar multipurpose plate reader.

Lee R: "Science of sailing", English Institute of Sports, 7th October 2008, XP055051667; URL:http://www.eis2win.co.uk/pages/news_scienceofsailing.aspx refers to a test using pholasin to measure chemiluminescence, which can provide information on whether treatment for the infection should begin, training continue or whether extra rest is necessary. The test involves only the measurement of chemiluminescence, without any lymphocyte count or neutrophil count.

There is a need for a technique which can determine objectively the physical condition of an athlete, preferably before peak condition is reached, so that the athlete may be maintained at peak condition and overtraining can be avoided.

An objective of the present invention is to provide an apparatus which, using an objective test based on a physiological sample from the subject, provides an indication of the physical condition of the subject, and optionally also provides recommendations for changes in the regime of the subject on the basis of that indication. For the avoidance of doubt, the subject may be a human (athlete, key worker, etc) or a competition animal such as a horse or dog.

A further objective, particularly in relation to human athletes, is to provide an apparatus capable of detecting the degree of overreaching and overtraining, and indicating changes in the training regime to return the athlete to peak condition.

A further objective, particularly in relation to human non-athletes, is to provide an apparatus capable of detecting overstressed individuals, for example individuals whose workload is excessive.

A further objective is to provide an apparatus capable of detecting infection in a subject, and optionally characterising that infection as viral or bacterial, before outward symptoms of infection are expressed in the subject. This may have utility in general medical practice, or in emergency room or pre-operative situations where detection of an infection may affect the care given to an individual.

The technique of the present invention makes use of particular combinations of data derived from analysis of a blood sample from a subject in order to give an indication of the "readiness to perform" of the subject which is more specific and informative than has hitherto been achieved.

According to a first aspect of the invention, there is provided a diagnostic apparatus according to claim 7. In one embodiment, the apparatus includes means to divide the blood sample into two parts, and the means for adding a chemiluminescent probe and the luminometer means for measuring light emitted operate on a first part of the blood sample, while the means for analysing the blood sample to generate a lymphoocyte count and the means for analysing the blood sample to generate a neutrophil count operate on a second part of the blood sample.

The means for adding a chemiluminescent probe to a first part of the blood sample may comprise a pre-filled reagent syringe, or may comprise a reagent reservoir and dosing means controllable to deliver reagent to a chamber containing a first part of the blood sample.

The means to record data representing light emitted from the said first part of the blood sample may produce an output in the form of a "luminescent curve" or "luminescence curve", which is a graphical trace showing the variation of the light emission over time. Alternatively, the data may be recorded simply as a series of values taken at known times. Data values may be recorded at regular intervals for a predetermined period following the addition of the chemiluminescent probe to the blood sample.

The result indicative of the physical condition of the subject may be expressed as a "readiness to perform" result. In the case of an athlete, the result may be expressed as "normal", "fatigued", "overreached" or "overtrained".

In one embodiment, the apparatus includes means for passing light through part of the blood sample, means for capturing an image, and means to generate lymphocyte and neutrophil count data from the captured image, means for adding a chemiluminescent reagent to another part of the blood sample, and means for detecting light emitted by that part of the blood sample over a period of time.

In another embodiment, the lymphocyte and neutrophil count data is generated by passing white blood cells from the blood sample between a pair of electrodes and measuring impedance between the electrodes at two differing frequencies, while the luminescence data is obtained by adding a chemiluminescent reagent to another part of the blood sample, and means for detecting light emitted by that part of the blood sample over a period of time.

A second aspect of the invention provides a diagnostic testing system according to claim 1. The means to record data representing light emitted from the blood sample may produce an output in the form of a "luminescent curve" or "luminescence curve", which is a graphical trace showing the variation of the light emission over time. In an alternative embodiment, the first network device may be replaced by two or more separate network devices, one of which may comprise means for analysing a blood sample from a subject to generate data representing a luminescence curve, while another network device may analyse a further blood sample from the same subject to generate a lymphocyte count, and a third network device may analyse a further blood sample from the same subject to generate a neutrophil count, and the separate network devices each include means to transmit the generated data over the communications network to the server (the second network device). It will be appreciated that more than one test can be carried out by each network device. Preferably the second network device includes means to transmit the notification over the communications network to the first network device, or to a third network device. The second network device may include means for receiving requests for information from remote network devices, and may provide notifications and/or test results in response to those requests.

A third aspect of the invention provides a server for use in the system, according to claim 4. In one embodiment, the data representing chemiluminescent light emission levels over a predetermined interval is in the form of a luminescence curve.

The server preferably includes means for determining that data representing a luminescence curve, a lymphocyte count and a neutrophil count has been received, and further includes a controller to initiate operation of the processing means when receipt of data for the generation of a fitness result has been determined.

The server preferably includes means to transmit the notification and/or the fitness result over a communications network to a remote terminal connected to the network.

Embodiments of the invention will now be described in detail with reference to the accompanying Figures, in which:
Figure 1 is a schematic perspective view, cutaway to show internal detail, of a portable diagnostic apparatus and its consumables, which comprise a cartridge, a blood sampling device and reagent syringes;
Figure 2 is a schematic sectional view of the apparatus with its cartridge inserted;
Figure 3 is a view of a pack of consumables for use with the diagnostic apparatus;
Figure 4 is a schematic view showing the internal components of the diagnostic apparatus;
Figure 5 is a diagram showing a first (normal) luminescence curve obtained from a luminometer measuring light emitted from a blood sample before and after the addition of reagents;
Figure 6 is a diagram showing a second luminescence curve, contrasted with the normal trace;
Figure 7 is a diagram showing a third luminescence curve, contrasted with the normal trace;
Figure 8 is a diagram showing a fourth luminescence curve, contrasted with the normal trace;
Figure 9 is a diagram showing fifth and sixth luminescence curves, contrasted with the normal trace;
Figure 10 is an example of a look-up table;
Figure 11 is a schematic perspective view, similar to Figure 1, of an alternative embodiment of the diagnostic testing apparatus;
Figure 12 is a schematic view of a cartridge for use in the apparatus of Figure 11;
Figure 13 is a schematic view of a second embodiment of the diagnostic apparatus;
Figure 14 is a schematic representation of the data formatter of the system of Figure 13;
Figures 15 is an example of the data structure in a subject database used in the system of Figure 13;
Figure 16 is an example of the data structure in a user database used in the system of Figure 13;
Figure 17 in a second alternative embodiment of a server for use in the system of Figure 13; and
Figure 18 is an example of a look-up table used in the second alternative embodiment of Figure 17.

### First Embodiment

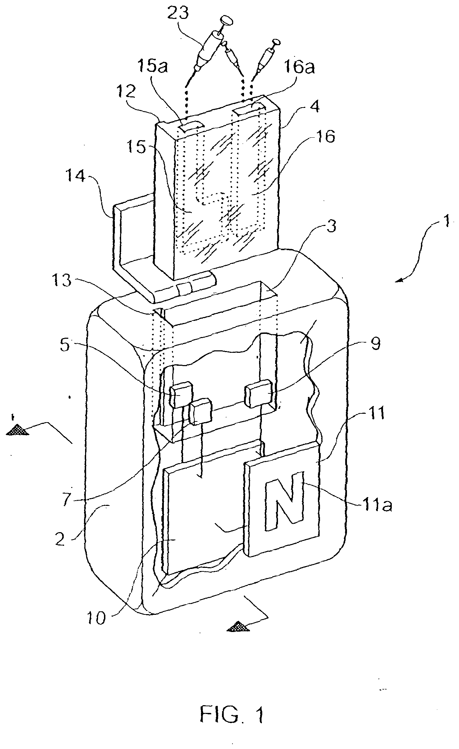

Referring to Figures 1 to 4, there is seen a first embodiment of the diagnostic apparatus, configured as a portable hand-held or table-top diagnostic device.

Referring to Figure 1, the diagnostic device 1 of the first embodiment comprises a generally rectangular body 2 having at its upper end a slot 3 to receive a diagnostic cartridge 4, which will be described in further detail below.

A light source 5 is positioned within the body 2 adjacent the slot 3, in alignment with a detector 7, so that light emitted by the source 5 is directed towards the detector 7. The detector 7 and may be a charge-coupled device capable of capturing an image, and imaging optics (not shown) may be provided to focus light from the source 5 at a focal plane within the blood sample. A microswitch 8 is positioned at the bottom of the slot 3, to detect the insertion of a cartridge 4 into the slot 3.

A luminometer 9 is also mounted within the body 2, adjacent the slot 3.

The emitters, detectors, microswitch and luminometer are connected to a circuit board 10, which is also connected to a display 11.

The cartridge 4 is made from transparent plastics or glass material, and the external dimensions of the cartridge are such as to fit closely within the slot 3 in the body 2 of the device. To ensure that the cartridge is placed into the slot 3 in the correct orientation, the cartridge 4 and slot 3 may have asymmetrical shapes. In the example shown, the cartridge 4 is formed with a rib 12 which cooperates with a groove 13 formed at the corner of the slot 3, so that the cartridge may only be inserted in the slot when correctly aligned.

The cartridge 4 has two internal cavities which open at the upper end of the cartridge (as seen in Figure 1). This end of the cartridge is exposed when the cartridge is placed within the device. In some embodiments, the body 2 may be provided with a hinged lid 14, optionally resiliently mounted, which closes over the top of the cartridge 4 when it is correctly mounted within the slot 3, and when closed may deny access to at least one of the cavities.

As seen in Figure 1, the cartridge has an "L" shaped cavity 15 on the left-hand side, and a generally rectangular cavity 16 on the right-hand side. The cavity 15 as an opening 15a on the top of the cartridge, while the cavity 16 has an opening 16a.

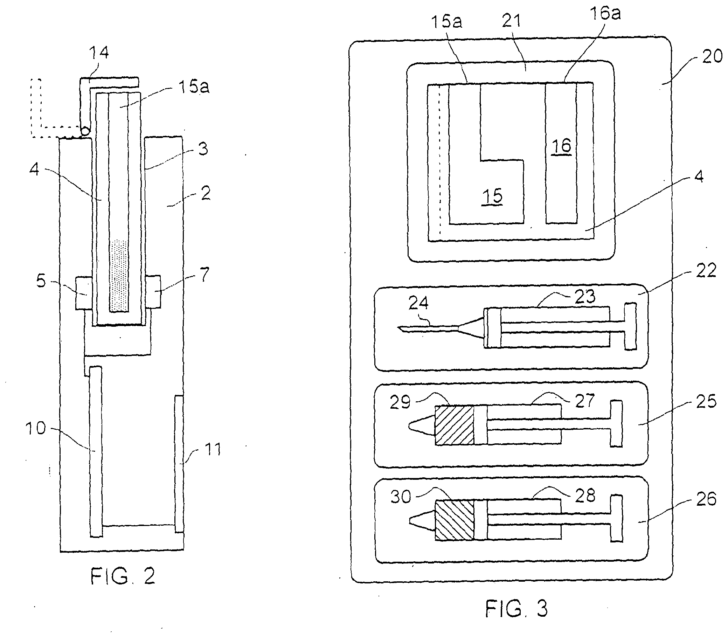

As may be seen in Figure 2, in the illustrated embodiment the lid 14 closes over the top of the cartridge 4 to deny access to the opening 15a when the cartridge 4 is mounted within the slot 3. Likewise, the alignment between the emitter 5 and detector 7 is illustrated in Figure 2. When the cartridge 4 is fully inserted in the slot 3, the lower, enlarged part of the cavity 15 extends between the emitter 5 and detector 7, and the lower end of the cavity 16 is adjacent the luminometer 9.

Figure 3 illustrates a package of consumables for use with the diagnostic apparatus of Figure 1. The package 20 may be formed as a sachet or a blister-type pack, in four distinct sections. Upper generally square section 21 of the blister pack contains a transparent cartridge 4 with cavities 15 and 16 open respectively at 15a and 16a. Cavity 15 is pre-loaded with a haemolysing agent and a staining agent. The haemolysing agent may be formic acid, and the staining agent may be methylene blue. The purpose of these reagents is to prevent the red cells from forming images in the detector, and to enhance the images formed by the white cells, respectively

Below the cartridge section of the pack, section 22 of the blister pack contains a sterile syringe 23 and needle 24, for taking a blood sample from the subject. Section 25 of the pack contains a first reagent syringe 27 pre-loaded with a first reagent 29 for adding to a part of the blood sample. Section 26 of the pack contains a second pre-loaded syringe 28, containing a second reagent 30.

To prepare the device for use, the pack 20 is first opened to remove the cartridge 4 and the syringe 23 and needle 24. Using the syringe 23, a blood sample is taken from the subject. This may be done from a vein in, for example, the hand or elbow. Sufficient blood is drawn so that the syringe may then be used to place part of the sample (through opening 15a) into the cavity 15 in the cartridge, where it mixes with the pre-loaded reagents, and to place another part of the sample into cavity 16 through opening 16a. A typical sample drawn from the subject will be from 5 to 10 ml of blood, of which up to about 5 ml may be placed in cavity 15 and up to about 3 ml may be placed in cavity 16. The cartridge may be provided with level marks (not shown) to indicate the amount of blood to be placed in each cavity.

The cartridge 4 is first aligned with the slot 3, with rib 12 and groove 13 in registration. The cartridge is then inserted fully into the slot 3 in the housing 2, and lid 14 is closed over the top of the cartridge to cover the opening 15a but leave opening 16a accessible.

The first reagent syringe 27 is then removed from the pack 20, and the contents of the syringe are added to the blood sample in cavity 16, through the exposed opening 16a. The amount of reagent 29 in the syringe 27 corresponds to the volume of blood in the cavity 16, so that the correct proportion of reagent is added to the blood sample. The first reagent 29 may be Pholasin (RTM), Lucigenin, Luminol, Isoluminol, 8-Amino-5-chloro-7-phenylpyrido[3,4-d]pyridazine-1,4-(2H,3H)dione(L-012), 2-Methyl-6[p-methoxyphenyl]-3,7-dihydroimidazo[1,2-α]pyrazin3-one (MCLA) or any other suitable photoprotein

After a predetermined time has elapsed, during which the luminometer establishes an initial value V0 for the light emission from the sample, the second reagent syringe 28 is removed from the pack 20 and its contents are also added to the mixture of blood and first reagent in cavity 16. Again, the amount of second reagent 30 pre-packed in the syringe 28 corresponds to the amount of blood and reagent mixture in the cavity 16. The second reagent is a trigger compound which stimulates emission of light, and may be N-formyl-Met-Leu-Phe (fMLP) or phorbol 12-myristate 13-acetate (PMA) or any other suitable chemiluminescent probe.

Instructions for the sequential addition of the reagents may be provided either by being printed on the pack, or may be provided on the display 11 of the device. The reagent syringes 27 and 28 and/or the pack 20 may be colour-coded, numbered, or otherwise marked to ensure that the reagents are added in the correct order.

Operation of the apparatus may be initiated by a sensor such as microswitch 8 within the housing 2 which detects when the cartridge 4 is fully inserted into the slot 3, or a switch linked to the lid 14 which detects when the cartridge 4 is fully inserted and the lid 14 is closed. Alternatively, a "start" button (not shown) may be provided on the housing 2 for the user to initiate operation once the cartridge has been filled and inserted.

When operation is initiated, the light source 5 emits light which shines through the blood sample held in cavity 15, and the detector 7 captures an image of the blood sample at a focal plane within the sample. A lens system (not shown) may be provided to bring the light to a focus at an appropriate plane within the sample cavity.

At the same time, the luminometer 9 starts to continually monitor the amount of light being emitted by the sample in cavity 16. This monitoring is continued over a period of time, generally about 1 to 3 minutes but possibly up to 5 minutes or longer, and the light emission level is periodically recorded, for example 5 times per second. Emission levels may be recorded more frequently, up to for example 20 times per second or more, or may be recorded less frequently down to for example once per second or less.

Circuitry on circuit board 10 within the device receives and processes the data from the detector 7, in order to produce a white blood cell count including at least a count of two particular types of white blood cells in the sample in cavity 15. The image processing may be based on the differential size of neutrophils and lymphocytes. The captured image may be processed to detect neutrophils, which are typically 10 to 12 µm in diameter, and lymphocytes, which are typically 7 to 8 µm in diameter, by conventional techniques for detecting generally circular darkened areas of these two particular sizes in the image. The cell counts are based on the number of cells of the particular sizes are detected in the captured image. The circuitry also receives and stores readings from the luminometer 9 over time.

The image processing circuitry may, in addition to discriminating neutrophils and lymphocytes, also produce accounts of other white blood cells on the basis of their size and population in the captured image.

The circuitry may cause an indication to be made on the display 11 prompting the user to add the first reagent 29 to the cavity 16. Monitoring of light emission continues after the first reagent is added. After an interval, the circuitry may cause the display 11 to prompt the user to add the second reagent 30 to cavity 16. Again, monitoring continues after addition of the second reagent until a predetermined testing period has elapsed.

In a simplified device, the user is not prompted to add the reagents by a message on the screen, but may simply be instructed by wording printed on the package 20 to fully insert the blood-filled cartridge 4, then wait, for example by counting up to 20, and then add the first reagent and then count again and add the second reagent. Since only one opening 16a of the cartridge is accessible, the user cannot add the reagent to the wrong cavity.

When the luminometer 9 has recorded light emission levels from the blood sample in cavity 16 for sufficient time, processing circuitry then computes a "readiness to perform" (RTP) and result for the athlete on the basis of the light emission levels and the blood counts, and this result is displayed on the display 11. In Figure 1, the display is an "N", indicating that the result of the blood analysis is that the athlete is an a normal training state. In the described embodiment, the display may indicate "N" for normal, "F" if the athlete is fatigued, "OR" if the athlete is overreached, or "OT" if the athlete is overtrained.

Once the result has been displayed, the apparatus may be prepared for a further use by removing and safely discarding the cartridge 4 and the three syringes. The next use will of course require a fresh pack 20 of consumables, in order to ensure that the blood sample is treated in a sterile environment with the correct amounts of reagents. Removal of the cartridge will release microswitch 8, which may cause the device to be powered off.

For an understanding of how the RTP result is reached, the interrelation between the blood cell counts and the variation over time of light emission from the sample mixed with chemiluminescent reagents will now be described.

### Characteristic luminescence traces

The testing of blood using a luminometer is an established practice, the results of the testing usually being expressed in the form of a graph called a "luminescence curve" plotting the amount of light emission against time. Recording of the emitted light is normally carried out over a period of approximately 350 seconds, and in the past the shape of the curve generated by plotting the luminescence values against time has been interpreted by professionals as a measure of neutrophil activity.

In each case, the light emission level of the mixture of blood sample and first reagent is first measured to establish an initial value V0. The second reagent is added, and the light emission level then rises at a gradient G1 to a peak value Vpeak. As time progresses, the light emission level falls at a gradient G2 to a final value VT at the end of the test period. Analysis of the shape of the light emission curve can give an indication of the physical condition of the subject.

### "Normal" trace

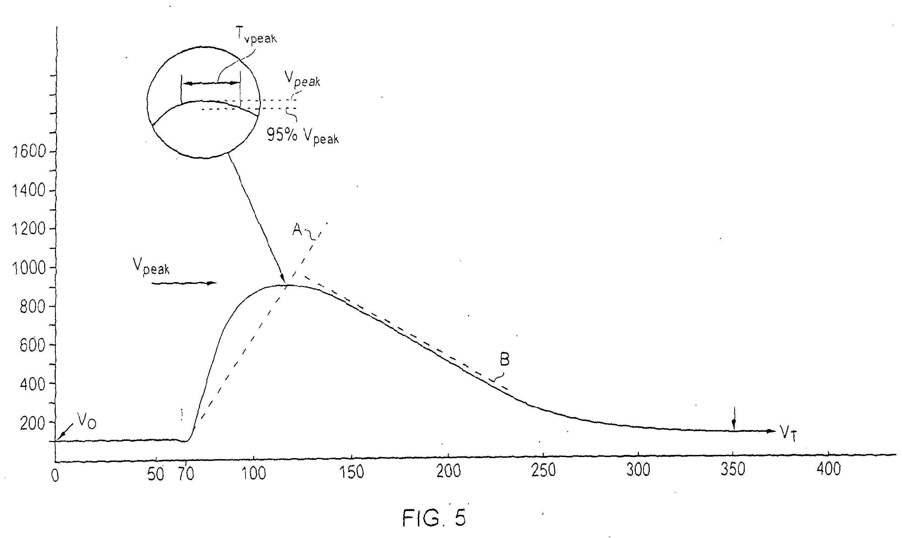

Figure 5 shows a "normal" trace. The luminescence measurement begins, with the first reagent (e.g. pholasin) added to the blood, and a light emission value V0 of approximately 100 and remains stable until the second reagent (e.g. fmlp) is added, at approximately t=60 seconds. The light emission value then increases up to a peak value VPeak of approximately 900 at about t=120 seconds and then reduces at a substantially constant rate until about t= and 250 seconds. The light emission value VT at the end of the test period (t=350 in this case) is measured at about 150 to 200. The luminosity values are typically recorded several times per second to generate the curve illustrated. In order to interpret the shape of the curve, measurements of the values V0, VPeak and VT are stored.

VPeak may be determined mathematically by subtracting from each reading the previous reading, and determining that when the subtraction renders a zero or negative result, VPeak has been reached.

The gradient G1 of the rising part of the curve which occurs after the addition of the second reagent may be calculated by differentiation, possibly taking a mean value of the gradient for that part of the curve between the time of addition of the second reagent and the time VPeak is reached. Alternatively, the gradient may be approximated by taking the time interval between the addition of the second reagent and the time at which VPeak is reached, and dividing it by the difference between the light emission values at those two times, so that the gradient G1 is represented by the slope of line A in Figure 5. In the example shown, the second reagent is added at t=70 when the light emission value is 100, and the peak value VPeak of 900 is reached at t=125, giving a value of +14.5 for gradient G1.

Similarly, the gradient G2 of the downslope following VPeak is of interest, and may again be calculated by differentiation. Alternatively, the gradient may be approximated by taking the values of the light reading at say 30 seconds and 80 seconds after VPeak to give a gradient represented in Figure 5 by the slope of line B in Figure 5. In the example shown, the light emission value decreases from 800 to 500 in the 50-second interval from t=150 to t=200, giving a value of -6 for gradient G2.

The "flatness" of the peak of the curve is represented by a time interval TVPeak which is derived from the light emission values by calculating the time for which the light emission value is within a predetermined proportion, say 5%, of VPeak. For the graph seen in Figure 5, the light emission value rises to VPeak and falls way quickly, so TVPeak will be a short interval of perhaps 1 to 2 seconds. A more rounded peak will have a value of TVPeak of perhaps 10 seconds or more.

The characteristics of interest in the luminescence curve are thus the initial value (V0), the gradient (G1) of the rising part of the curve, the magnitude of the peak of the curve VPeak, the roundness the peak of the curve represented by TVPeak, the gradient (G2) of the falling part of the curve, and the final value (VT) of the light emission reading.

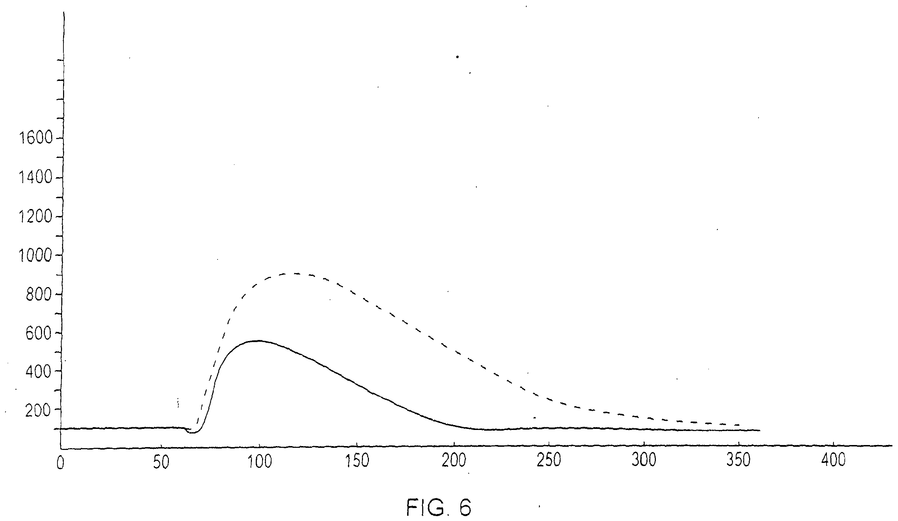

Different states of physical condition of the athlete subject produce different shapes of curve from the luminometer results. These are shown in Figures 6 to 9 with the normal trace of Figure 5 shown as a dashed line by way of comparison.

### "Fatigued" Trace

Figure 6 shows a "fatigued" trace, from a subject who is slightly below his optimal performance. The characteristics of this curve are that it rises with a slope similar to the normal trace, but reaches a peak at a much lower value than the normal trace. After a distinct peak, the trace has a downslope gradient similar to that of the normal trace, returning to the final value at an earlier time than the normal trace. In the example shown in Figure 6, the initial light emission value V0 is again 100 and remains stable until the second reagent is added, at approximately t=60 seconds. The light emission value then increases up to a peak value VPeak of approximately 600 at about t=90 seconds, to give a rising gradient value G1 of 16.6, and then reduces at a substantially constant rate until about t=200 seconds, after which it remains stable at a value of 150. This results in a downslope gradient G2 of -4. The light emission value VT at the end of the test period (t=350 in this case) is measured at 150. The "flatness" of the peak is represented by the time interval TVPeak for which the light emission value is within 5% of VPeak, i.e. the interval in which the value is above 570. In the trace of Figure 6, this interval is less than 2 seconds.

### "Overreached" Trace

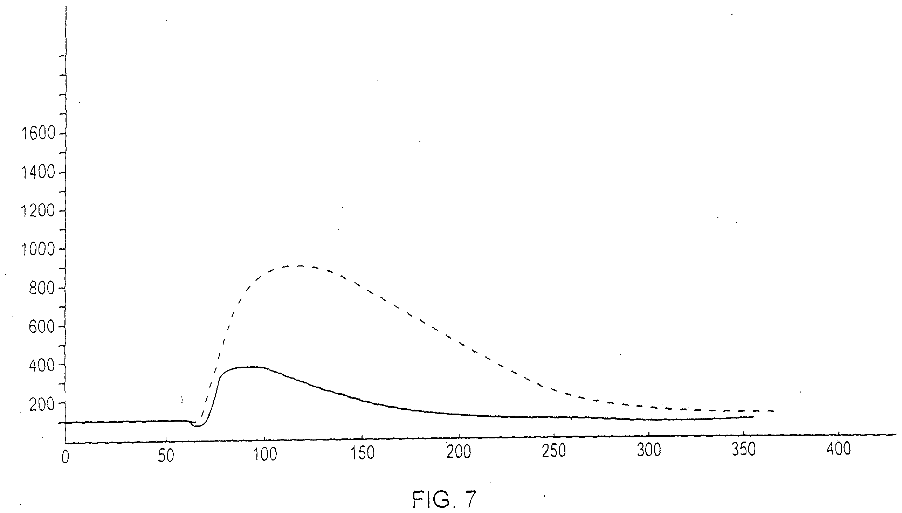

Figure 7 shows an "overreached" trace, from an athlete who is more noticeably below his optimal performance. The characteristics of this curve are that it rises with a slope similar to the normal trace, but reaches a rounded peak at a very much lower value than the normal trace. After the rounded peak, the trace has a downslope gradient less steep than that of the normal trace, returning to the final value at an earlier time than the normal trace. In the example shown in Figure 7, the initial light emission value V0 is again 100 and remains stable until the second reagent is added, at t=60 seconds. The light emission value then increases up to a peak value VPeak of approximately 400 at about t=80 seconds, to give a rising gradient value G1 of 15. The value remains at about 400 for approximately 20 seconds, and then reduces at a substantially constant rate until about t=200 seconds, after which it remains stable at a value of 150. This results in a downslope gradient G2 of -3.5. The light emission value VT at the end of the test period is measured at 150. The "flatness" TVPeak in this case is about 25 seconds.

### "Overtrained" Trace

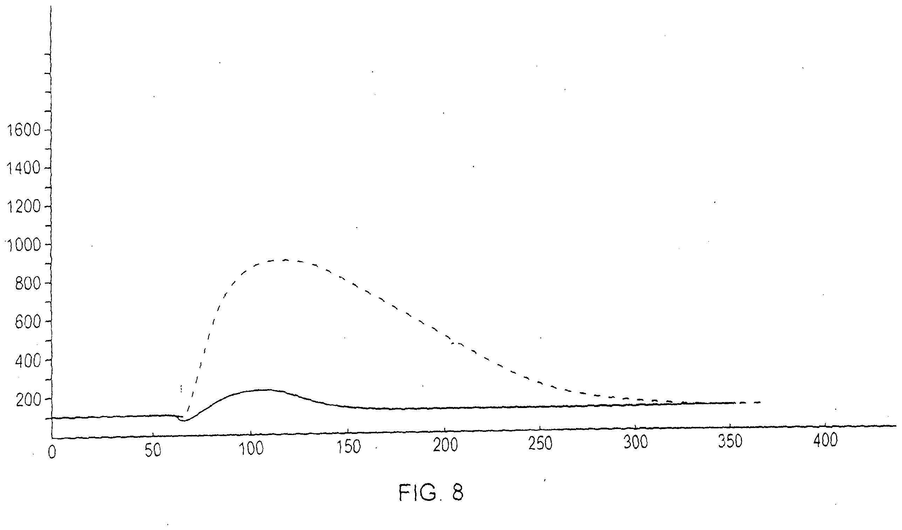

Figure 8 shows an "overtrained" trace, from an athlete who is so far below his optimal performance as to be completely exhausted. In this condition, the athlete's performance will be severely limited and it will take a long period, possibly months, of rest for him to recover. The characteristics of this curve are that it rises with a slope much less steep than the normal trace, and reaches a rounded peak at a very much lower value than the normal trace. After the rounded peak, the trace has a downslope gradient nearly symmetrical to its upslope, and much less steep than the normal trace. In the example shown in Figure 8, the initial light emission value V0 is again 100 and remains stable until the second reagent is added, at t=60 seconds. The light emission value then increases up to a peak value VPeak of approximately 200 at about t=110 seconds, to give a rising gradient value G1 of 2. The value remains at about 200 for approximately 15 seconds, and then reduces at a substantially constant rate until about t=160 seconds, after which it remains stable at a value of 150. This results in a downslope gradient G2 of -2. The light emission value VT at the end of the test period is measured at 150. The "flatness" TVPeak in this case is 15 seconds.

### Detection of Infection

In addition to detecting the training state of an athlete, the luminescence curve data may be used, in conjunction with the blood count results, to detect bacterial and viral infections, even before the subject exhibits any outward symptom of infection.

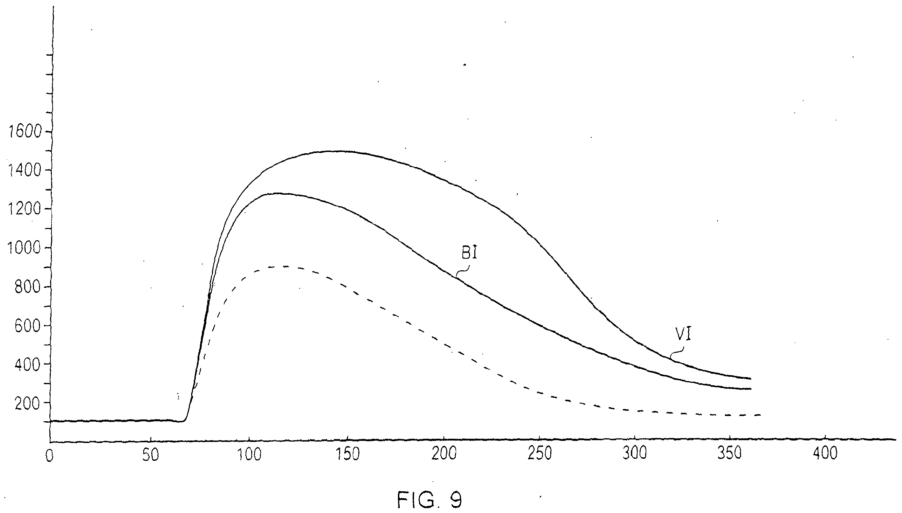

In the case of an infection, the curves generated by the luminescence test are illustrated in Figure 9.

Curve VI exhibits a steep initial upslope gradient G1 of about 20, and rises to a very high peak value VPeak of about 1400, and then remains at a high value for a considerable period (TVPeak is typically over 10 and up to 50 or more seconds) before falling rapidly to a final value of approximately 400, with a downslope gradient of -20. This characteristic shape of curve indicates the likelihood of a viral infection, and by combining the curve characteristics with the blood count results, the presence of a viral infection can be determined. A viral infection is indicated when the lymphocyte count is elevated above the subject's normal value and is greater than the neutrophil count.

Curve BI of Figure 9 exhibits a steep initial upslope gradient G1 of about 20, and rises to a very high peak value VPeak of about 1200, and then remains at a high value for a considerable period (TVPeak is over 10 seconds) before falling gradually to a final value of approximately 400, with a downslope gradient of -6.

This characteristic rounded shape of curve indicates an infection, and by combining the curve characteristics with the blood count results, the presence of a bacterial infection can be determined. When the neutrophil count is elevated above the subject's normal value and is greater than the leukocyte count, a bacterial infection is indicated.

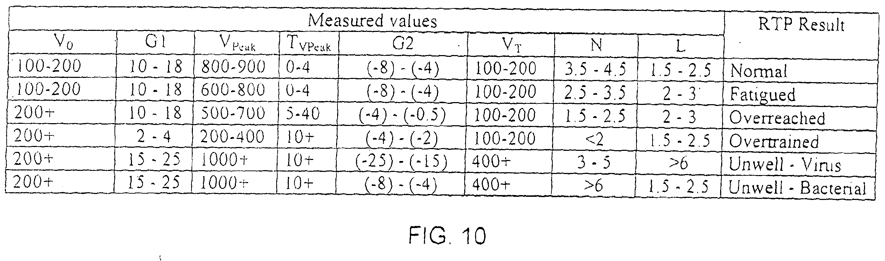

In the lookup table of Figure 10, the bottom two lines of the table correspond to ranges of curve parameters characteristic of infection, and when taken in conjunction with the blood counts lead to a result which indicates that the athlete is unwell, with either a viral or a bacterial infection.

In the white blood cell count method described above, part of the whole blood sample is treated with a lysis solution to destroy the red blood cells, and a stain to enhance the appearance of the white blood cells prior to capturing an image of a part of the sample. Image processing circuitry in the apparatus then detects in the image regions which correspond to stained white cells of the characteristics sizes of neutrophils and lymphocytes, and counts them.

The characteristics of the light emission curve are measured and/or calculated, and are combined with the blood count results to produce an indication of the training condition of the athlete.

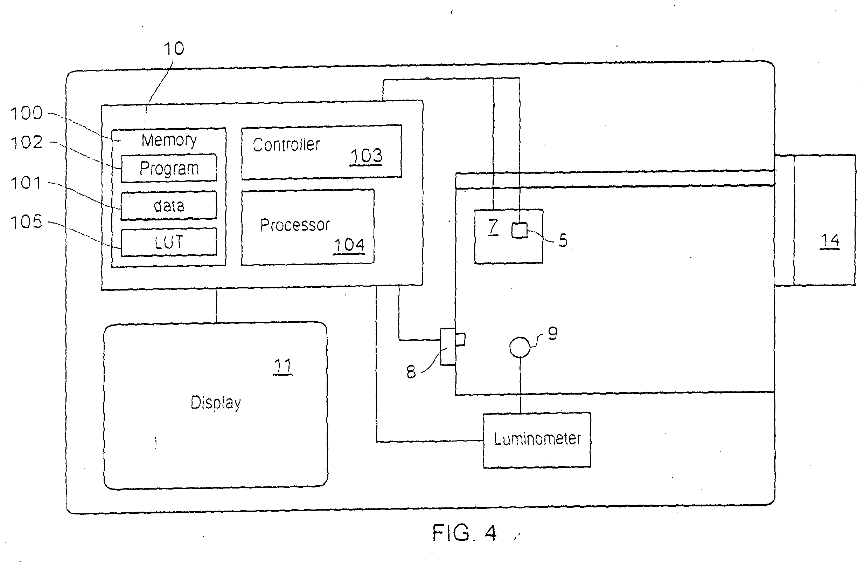

Referring now to Figure 4, circuit board 10 comprises a memory 100 which stores data received from the luminometer 9 and the detectors 7 and 8 in a data file 101, and stores program data in a program file 102. A controller 103 executes the instructions in program file 102, to control the storage of data from the detectors in the data file 101, and to control the supply of data from the data file 101 to a processor 104 to make the calculations necessary to establish the values of the curve parameters V0, G1, VPeak, G2 and VT.

The memory 100 also stores a look-up table 105, whose data structure is illustrated in Figure 10. In the look-up table, ranges of values for the curve parameters and ranges of neutrophil and lymphocyte counts which correspond to each of six "Readiness to Participate" (RTP) results. The values shown in the lock-up table in Figure 10 are of course exemplary, and values such as gradients will depend on the units of measurement used to detect the level of light emissions in the luminescence test. Further, individual subject may have different ranges of "normal" readings for blood cell counts, and this will give rise to different ranges of data to be used in look-up tables tailored specifically to those subjects.

The apparatus of the described embodiment compared the calculated and measured values of the curve parameters and blood counts with the values in the lookup table, and determines the corresponding RTP result. The result is then displayed on the display 11.

This result is then displayed as the RTP result of the athlete on display 11 as "UB" or "UV" for bacterial or viral infections, respectively.

### Alternative Variants

In order to establish a baseline value for the determination of the blood counts, as a first step in the testing process, the cartridge 4 may be inserted into the apparatus empty, and the light source 5 operated so that the detector 7 detects an image caused by the cartridge alone. The cavities 15 and 16 in the cartridge 4 may then be filled, possibly with the cartridge still in the apparatus, and a second image may be captured by the detector 7. Subtracting the pixel values of the first image from corresponding pixels in the second image eliminates false readings caused by the cartridge material. By performing the calibration each time the apparatus is used, differences in the light transmission characteristics of different cartridges can be taken into account in the final detection result.

In the embodiment described above, syringes are used to manually add reagents to the chamber 16. In an alternative variant of this embodiment (not illustrated), the housing is provided with two reservoirs each adapted to contain a quantity of the respective reagent, and dosing means are provided to operate under the control of the apparatus to provide predetermined amounts of the reagents at appropriate times for the luminosity data to be recorded. In this variant, the consumables pack contains only a sterile cartridge and a sterile syringe for obtaining a blood sample. When the sample has been placed in the cavities in the cartridge, the cartridge is inserted into the apparatus and the controller operates the dosing means to add the required quantities of the reagents to one of the cavities at the appropriate times for the luminosity testing operation to be completed. In other respects, the apparatus may operate as described above.

In variants of the apparatus where a smaller blood sample is required, the syringe 23 may be replaced by a stylet which simply pierces the subject's skin so that a drop of blood can be collected or deposited directly into the cartridge.

When a subject has undergone a series of tests which lead to the calculation of RTP results, examination of the subject's actual physical condition and comparison of this condition with the RTP results may allow the adjustment of values in the look-up table so as to tailor them to the individual subject.

One or more additional parameters, such as a measurement of the area under the luminescence curve, measurement of the time taken between adding the reagent and reaching the peak value VPeak, measurement of the width of the curve at half the maximum value (i.e. the time interval between reaching half VPeak on the rising part of the curve and again reaching half VPeak on the falling part of the curve), and/or measurement of the "activation increase" which is the difference between the peak value VPeak and the base value V0, may also be factored into the look-up table and serve as a basis for determining the "readiness to perform" result.

In a further alternative embodiment of the apparatus, the white blood cells may be counted by a conventional optical analysis technique in which a reagent is first added to the blood sample so as to cause a white cell peroxydise reaction. The sample is then passed through a beam of white light and sensors detect light scattered at a narrow (2 to 3°) angle, and light scattered at a wider (5 to 15°) angle, respectively. Processing circuitry then plots light scatter against light absorbent is to generate measurement of the number of neutrophils, monocytes, eosinophils and lymphocytes.

### Inductive Method for White Blood Cell Count

In an alternative apparatus, the white blood cell count values for neutrophils and lymphocytes may be obtained electrically, rather than optically as described above.

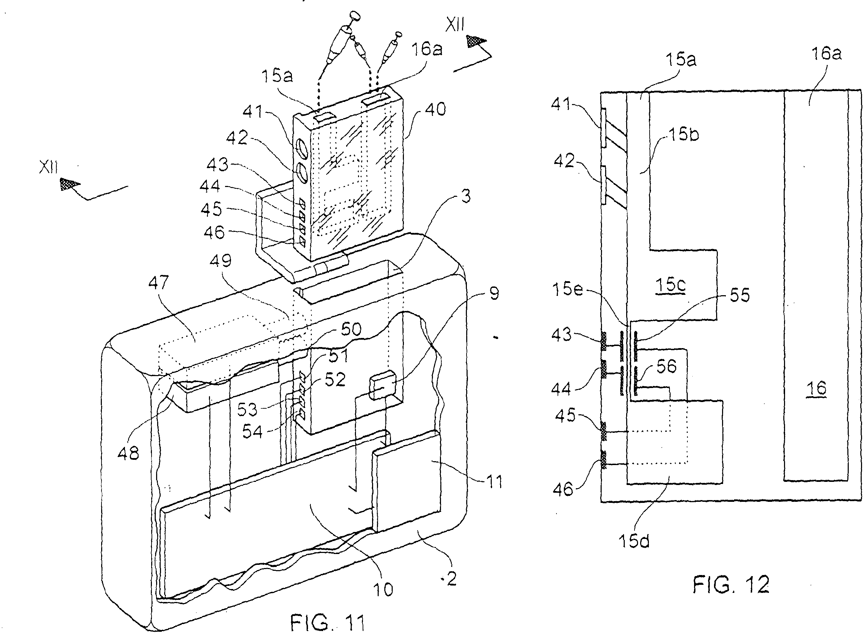

Figures 11 and 12 illustrate an apparatus in which the white blood cell count values for neutrophils and lymphocytes are obtained electrically. The 11 is a view of the apparatus similar to Figure 1, and Figure 12 is a schematic sectional view of the cartridge in the plane XII-XII of Figure 11.

Referring now to the Figures, the apparatus comprises a housing 2 having a slot 3 into which the cartridge 40 is receivable. The apparatus as before comprises a display 11, a circuit board 10 and a luminometer 9.

The cartridge 40 includes 2 dosing ports 41 and 42 on the upper part of its left-hand side (as seen in the Figure), and on the lower part of the left-hand side there are four electrical contacts 43 to 46.

Within the housing 2 of the apparatus, two reagent reservoirs 47 and 48 are provided, each reservoir having a respective dosing device 49 and 50 for delivering a quantity of reagent from the reservoir 47, 48 to a respective one of the dosing ports 41, 42 when the cartridge 40 is inserted into the slot 3 in the housing 2. The dosing devices of 49 and 50 controlled by circuitry on the circuit board 10.

On the side of the slot, electrical contact pads 51 to 54 are provided, positioned so as to contact respectively the electrical contacts 43 to 46 of the cartridge 40, when the cartridge 40 is inserted into the slot 3 in the housing 2. The contact pads 51 to 54 are connected to circuitry on the circuit board 10.

Figure 12 is a schematic sectional view, to an enlarged scale, of the cartridge 40. As before the cartridge has openings 15a and 16a on its upper face, the opening 16a leading into a cavity 16 which is aligned with the luminometer 9 within the housing 2 when the cartridge 40 is inserted into the slot 3. The cavity 16 is used in the same way as previously described, to add reagents to a part of the blood sample and record light emissions from the sample over a period of time to generate the "luminescence curve" data.

On the left-hand side of the cartridge, as seen in the Figure, the opening 15a leads into a first passage 15b which opens into a mixing chamber 15c. Dosing ports 41 and 42 are connected to the first passage 15b, so that reagents delivered by the dosing devices 49 and 50 enter the dosing ports 41 and 42 and are led via the first passage 15b to the mixing chamber 15c.

Below the mixing chamber 15c is a waste chamber 15d, linked to the mixing chamber via a measuring passage 15e. Two pairs of electrodes 55 and 56 are positioned in the cartridge such that the measuring passage 15e passes between the electrodes of each pair. Each electrode is connected to a respective contact pad on the side of the cartridge.

In operation, as before a blood sample is taken from the subject and part of the sample is introduced through opening 15a into the mixing chamber 15c, while another part of the sample is introduced through opening 16a into the chamber 16.

The sample blood in the chamber 16 is treated as previously described by adding reagents at intervals, and recording light emission from the sample with luminometer 9.

When the cartridge 40 has been inserted into the slot 3, the electrical contacts 43 to 46 link the electrode pairs 55 and 56 to the circuit board 10, which provides alternating voltage to the electrode pairs at different frequencies. Electrode pair 55 may be supplied with automating voltage at a frequency of about 0.5 MHz, while electrode pair 56 is supplied with voltage at about 1.7 MHz.

The circuitry 10 includes a controller which controls the operation of dosing device 49 to add a haemolysing
reagent such as formic acid from the first reagent reservoir 47 to the blood sample in mixing chamber 15c via the dosing port 41. After a delay, during which the haemolysing agent disrupts the red blood cells in the sample, a quench solution is dosed from the second reagent reservoir 48 by the dosing device 50 and dosing port 42 to return the sample to an isotonic state. The quench solution may be a saline solution containing sodium carbonate.

The sample in the mixing chamber 15c, containing intact white blood cells, then passes through the measuring passage 15e and between the pairs of electrodes 55 and 56. The measuring passage 15e is so die mentioned that the larger white blood cells are constrained to pass through the channel one at a time. The diameter of the measuring passage is preferably about 40µm. Alternatively, the measuring passage may be a square or rectangular-section channel with transverse dimensions in the range of 40µm to 50µm. The mixture may pass through the measuring passage under gravity, by capillary action, or may be driven through the passage by a pump (not shown) mounted to the cartridge and powered and controlled by the circuit board 10 via electrical connections.

As the white blood cells pass between the electrodes, circuitry in the circuit board 10 measures the impedance between the electrode pairs. As the white blood cells pass, a change in the impedance is caused by each passing cell. By measuring the change in impedance at two different frequencies, different types of white blood cells can be discriminated and a count of neutrophils and lymphocytes may be derived from the impedance results. In one embodiment, impedance at each of the two different frequencies is measured as each cell passes through the measuring channel. The impedance measurement at 500 kHz gives an indication of the volume of the cell passing between the electrodes, while the ratio of the impedances at 500 kHz and 1.7 MHz effectively measures membrane capacitance properties. By plotting, for each cell, a graph of the ratio of the two impedances against the impedance at 500 kHz for each passing cell, different varieties of white blood cells can be discriminated and counted.

Circuitry on the circuit board 10 measures the impedance across the pairs of electrodes, and for each event where the impedance changes, calculates the ratio of the two impedance changes and characterises the cell passing through the electrodes as a neutrophil or a lymphocyte on the basis of the impedance change at 0.5Mhz and the ratio of the impedance changes. The number of cells passing through the measuring passage in a predetermined amount of time, in conjunction with the flow rate of sample through the passage, gives an indication of the cell counts in the blood sample.

Having obtained the cell counts and the "luminescence curve", the circuitry then proceeds to generate a "readiness to perform" result in the manner described above, by comparing the calculated values with values stored in a look up table, and presents the result on the display 11.

In a yet further alternative embodiment, the whole blood sample may be passed through the measuring channel, without first lysing the red blood cells, and by measuring the variations in impedance a count of red blood cells and platelets, as well as white blood cells, may be determined by the processing circuitry.

To prepare the device for further use, the cartridge is removed and discarded and a check is made that the reagent reservoirs 47 and 48 still contain sufficient reagent for the next operation.

The reagent reservoirs 47 and 48, with their respective dosing devices 49 and 50, may themselves be removable from the housing 2 when empty, for replacement with full reservoirs. The circuitry on circuit board 10 may cause an indication to be given on display 11 when the levels of reagents in the reservoirs 47 and 48 are so low as to require refilling or replacement of the reservoirs.

In a further embodiment, not illustrated, the cartridge may be provided with dosing ports connected to cavity 16, and the housing may contain further reagent reservoirs storing the reagents for use in the photoluminescence test, together with control circuitry to ensure that the correct amounts of reagents are added at the correct intervals to perform the luminescence testing. In such an apparatus, the subject needs only to take a blood sample and place some of the sample in each of the 2 cavities in the cartridge, and then insert the cartridge in the slot 3. The circuitry 10 will then control the addition of reagents to the parts of the sample, and collect the data from the sensing devices to calculate the fitness result. It is also foreseen that, in a further variant of the device, the subject will place a blood sample in the apparatus and the apparatus will include means to divide the sample into parts for undergoing the separate testing techniques discussed above. For example, the apparatus may include an initial receptacle into which the blood is placed by the subject, and from which capillary tubes lead parts of the blood sample to respective testing areas in the device.

### Second Embodiment

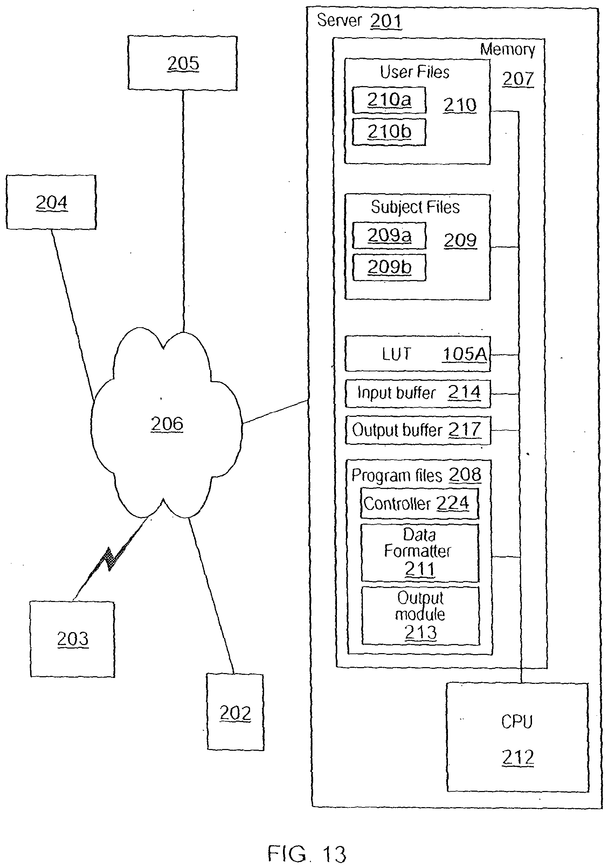

Figures 13 to 18 illustrate a second embodiment of the invention. In this embodiment, the analysis of blood samples to determine the training condition of an athlete may be performed at remote specialist facilities such as a pathology laboratory rather than by the athlete himself. The system further provides for the collection and collation of other data relating to the athlete's training condition which can be incorporated into the calculation of an RTP result. This additional data may be provided by observing the athlete at a training facility, from the athlete's medical advisers and/or trainers, or from the athlete himself. Users of the system may commission tests to be carried out in remote laboratories and the results sent to the system server, where RTP results are determined from the test data. Users may then download the RTP results data from the system server.

In this embodiment, a server 201 is connected to a number of terminals 202, 203, 204 and 205 via a communications network 206 such as the Internet.

Terminal 202 may, for example, be a personal computer or workstation. Terminal 203 may be a portable device such as a smart phone, capable of sending data in the form of e-mail or text messages. Terminal 204 may be a computer terminal in a pathology testing laboratory, coupled to an automated testing apparatus capable of performing automated testing of, for example, blood or urine, the terminal 204 being capable of receiving the test results and transmitting the test results over the network to the server 201. Terminal 205 may be a hand-held blood analysis device such as described earlier, additionally provided with means to connect the device to the communications network and transmit blood analysis data over the network to the server 201. The communications apparatus may be built into the blood analysis device, or the device may be provided with an external data port which is connectable to a communication device such as a modem or a personal computer, or a mobile device such as a smart phone, for sending data over the network.

### The Server

The server 201 comprises a memory 207, which includes a program memory 208 for storing program files, a subject database 209 which stores data on subjects (athletes or animals) in individual data files 209a, 209b, etc, a user database 210 which stores data on users of the apparatus in respective user files 210a, 210b, a look-up table 105A, and input buffer 214 and an output buffer 217.

In the program memory 208, there is stored a data formatter module 211 which receives incoming data from the communications network 206, and arranges the data in appropriate formats for storage in the subject files 209a, 209b and user files 210a, 210b, and an output module 213 to produce output messages either on the basis of incoming test data, or on request from user terminals.

The server further includes a processing unit 212 which processes the data to produce RTP results for respective athletes.

### Data Formatter

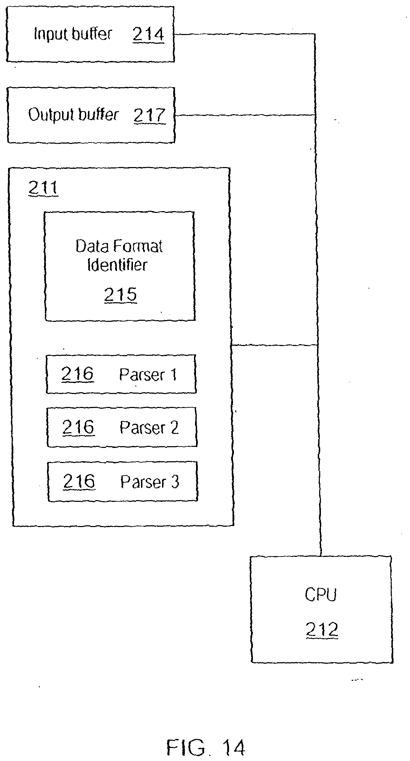

Figure 14 illustrates the structure and operation of the data formatter 211. Incoming data files received at the server from the communications network 206 are initially stored in the input buffer 214. Data files are then sequentially sent to the processor 212 under control of the data format identifier program 215. The processor determines the format of the incoming data, for example by examining the file name and determining the format on the basis of the file handle (.doc, .csv, .docx, etc). On the basis of this determination, the data file is then processed using one of a number of parser programs 216. Each parser program causes the processor 212 to convert data from a determined first file format into the format required for storage in the user files or subject files in the memory 207. The formatted data is then sent to an output module 217, which interrogates the data to determine which subject or user the data relates to, and directs it to the appropriate data file in the subject or user database. The formatted data is then added to the data in the appropriate data file. The data format identifier may receive a notification when a data file corresponding to a set of blood analysis results is received, and may automatically retrieve the results from the input buffer, format them and store them in the output buffer. The formatter 211 may then retrieve the formatted data from the output buffer 217 and store it in the appropriate subject or user file.

### Subject Database

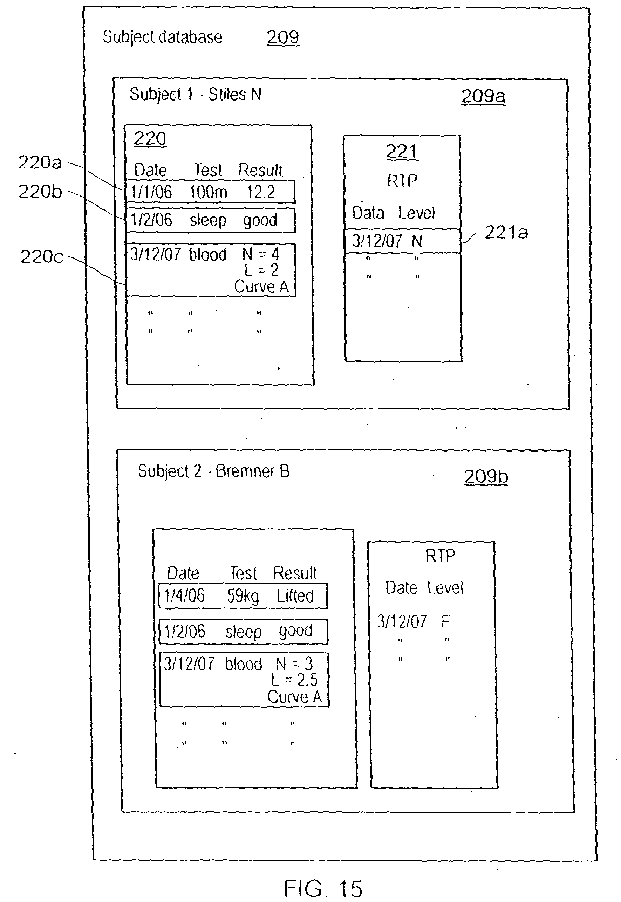

Figure 15 illustrates the typical types of data stored in the subject database 209. For each individual athlete, details of test results are recorded, with the date, the nature of the test, and the results of the test stored in a test results table 220. In the example shown in Figure 15, result 220a in the test results table 220 indicates the athlete "Subject 1" recorded a time of 12.2 seconds to run 100m on 1 January 2006. Result 220b indicates that on 1 February 2006 a subjective test on the quality of the athletes sleep gave the result "good". Result 220c indicates that on 3 December 2007 a blood test was made in which the athlete recorded a neutrophil count of 4, a lymphocyte count of 2, and a set of data A for the luminescence curve.

Subject file 210a also includes a record 221 of RTP results which are calculated from the blood test data by the processing unit 212. In the RTP result 221a, it is seen that the result "normal" was calculated from the blood test results received on 3 December 2007.

The subject file may also include one or more look-up tables tailored to the individual subject, for use in calculating the RTP results on the basis of a subject's blood tests. Such look-up tables may include further parameters on which the determination of an RTP result is made, including parameters such as the measured area beneath the luminescence curve generated by a blood test.

A second subject file 210b records similar data and RTP results for the athlete "Subject 2". As will be appreciated, each athlete "Subject n" using the system will have his or her own subject data file 209*n.*

### User Database

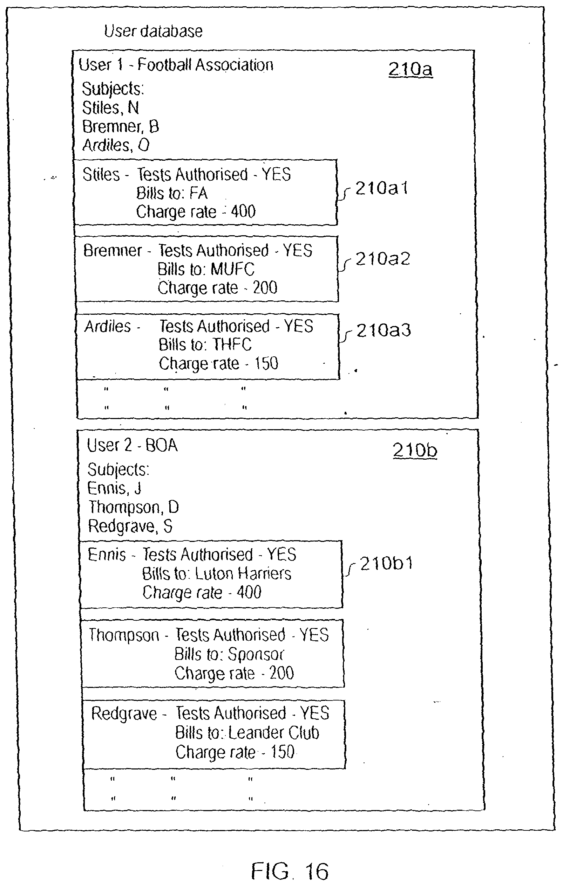

Figure 16 illustrates an example of the data stored in a user database 210. In this example, each "user" corresponds to a sport governing body, which is responsible for the performance of a number of subjects, and the user data file 210a will list the subjects associated with that user, and store details in a data file 210a1, 210a2, 210a3, etc relating to each of the athletes associated with that user. The data may include information such as whether the user authorises tests to be performed on the athlete, and other details such as who will have access to the test results for that athlete, who will bear the cost of the tests, how the user and/or the athlete should be contacted (for example e-mail, text message) when new test data are available.

### Operation

In this embodiment, the server includes a look-up table 105A similar to that illustrated in Figure 10, a processor 212 for performing calculations and a controller program 224 for controlling the flow of data.

When blood test results are received at the server 201, the controller 224 causes the data to be stored in the input buffer 214. Data formatter 211 then retrieves the data from the input buffer and reformats it into the appropriate form to be added into the relevant user or subject file. The controller 224 retrieves the blood test results from the data file, and provides them to the processor 212 which calculates the luminescence curve parameters as described in relation to the first embodiment, and compares the calculated parameters and blood count data with the look-up table 105A to determine an RTP result. The controller then stores the RTP result in the appropriate subject data file.

If the subject's data file includes a tailored look-up table, the tailored look-up table is retrieved from the subject's file 209a, b, etc, along with the formatted test data, and the processor 212 compares the test data with the tailored look-up table to determine an RTP result. The controller then stores the RTP result in the appropriate subject data file.

When a new RTP result is stored in a subject data file 209a, the controller 224 may retrieve from the user file database 210 the identity of the user associated with that subject, and cause the output module 213 to prepare and dispatch a notification, by means of for example an e-mail, text message or voicemail, to the relevant user and/or to the subject. The notification may include the new RTP result derived from the blood test, or may simply be a notification that a new test result for that athlete has been added to the database.

The controller 224 may cause the output module 213 to prepare and dispatch a notification to the relevant user and/or the relevant subject whenever any new test result for that subject has been added to the database, such as whenever the athlete has completed a physical test or a subjective assessment such as a sleep evaluation, and the result of the test or assessment has been communicated via the communications network to the server 201.

### Alternative Embodiment

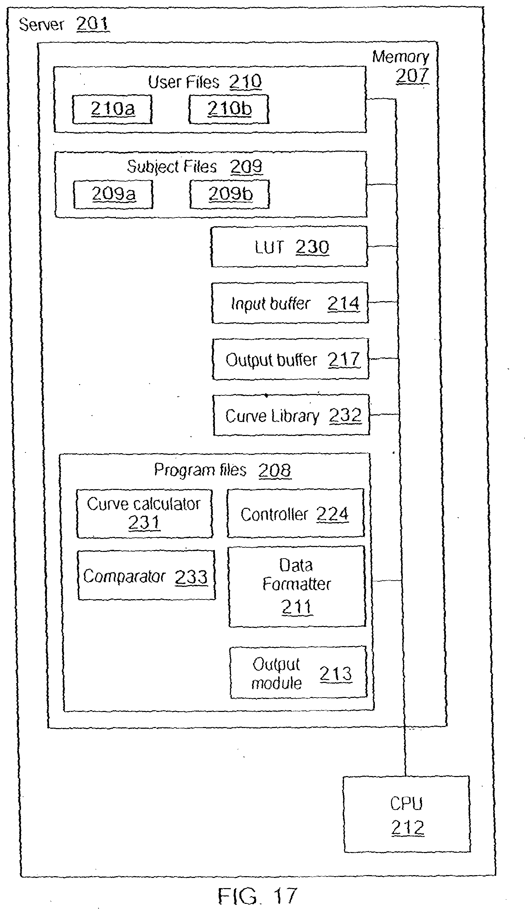

An alternative arrangement for the server 201 is illustrated in Figure 17. In this arrangement, the memory 207 additionally includes a curve library 232 storing characteristic profiles of luminescence curves, and the look-up table 230 differs from the previously-described table. The program memory 208 additionally includes a curve calculator 231 and a comparator 233. The controller 224 and a processor 212 are as described in relation to the embodiment of Figure 15.

In this embodiment, when blood test data representing a luminescence curve is received at the server, the.data is sent to the input buffer 214, formatted as before by the data formatter 211, and stored in the relevant subject file 209a. Controller 224 retrieves the test results and, using the curve calculator program 231, the processor 212 derives the shape of the curve from the test data.

### Curve Library

The curve library 232 stores six model curves, which will be referred to as curves A to F. These curves correspond to the shapes of curve seen in Figures 5 to 9. Curves A to D (corresponding to Figures 5 to 8, respectively) correspond to the characteristic luminescence curve shapes for normal, fatigued, overreached and overtrained athletes, and curves E and F (corresponding to the curves VI and VB of Figure 9) correspond to the characteristic curve shapes for athletes suffering from viral and bacterial infections, respectively.

### Comparator

The calculated shape of the curve is compared with the curves A to F in the curve library 232 by the processor 212 under control of the comparator program 233. The comparison may be made by any conventional mathematical technique, such as for example a "least-squares" method. Commercially available software such as "R" or numerical software such as GNU Scientific Library, SciPy and OpenOpt may be used to perform the curve comparisons.

The result of the comparison is to determine which of the model curves A to F most closely resembles the test results. The determination is then returned to the processor to be combined with the neutrophil and lymphocyte count data. These three values are then compared with values in the look-up table 230, to determine an RTP result.

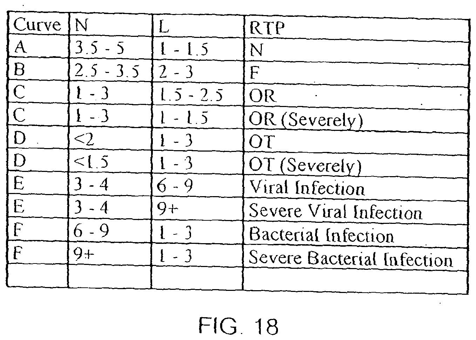

Figure 18 illustrates the structure of the lookup table 230. The table has, for each combination of curve shape and blood cell count values, a corresponding RTP result.

When the RTP result has been determined, the controller then stores the RTP result in the appropriate subject data file, as previously described. The controller 224 may, as before, retrieve from the user file database 210 the identity of the user associated with that subject, and cause the output module 213 to prepare and dispatch an e-mail, text message or other notification to the relevant user and/or to the subject conveying the RTP result derived from the blood test.

### Further alternative Embodiments

In the above-described embodiments, the RTP result has been derived from the blood test data by means of look-up tables. It is envisaged that, as an alternative to a look-up table, a processing algorithm may be used to directly determine an RTP result from the input luminescence data and blood cell count data. In such an algorithm, the RTP result may be determined from not only luminescence data and white blood cell count data, but also taking into account other sources of data such as urine tests, performance tests etc.

### Additional Athlete Information

In addition to the blood test data stored in the subject data files 209a, b etc, the subject data file may also store data relating to an athlete's performance.

For example, an athlete may perform a test exercise, such as running 100m, and the test exercise may be timed. Using a smart phone 203 or other user terminal, a user may connect to the server 201 and download a form in which the athlete's name, the date, the nature of the test and the performance data can be entered. The user may then fill in the relevant data on-screen, and transmit the form as a data file over the communications network 206 to the server 201, where the data formatter 211 will identify the data file, format the data, and store the data in the relevant subject file.

In another example, a blood testing laboratory may receive a blood sample from the athlete, and may perform for example a full blood count and a luminescence test on the blood sample. The blood testing machine may be connected to a computer terminal 204 in the laboratory, so as to provide the blood test results data to the terminal 204. Software in the terminal 204 may then place the data into a data file for transmission over the Internet to the server 201, where the data formatter 211 will convert the data (if necessary) and store the data in the relevant subject file. The luminescence data and blood counts required to determine the RTP result will then be retrieved from the data file and forwarded to the processor as previously described, so that an RTP result can be calculated and communicated to the athlete.

Access to the data stored in subject files may be made available over the Internet to a user terminal 202. A user of terminal 202 may input a request for data concerning a particular subject. When the request is received at server 201, a program stored in the program memory 208 will send a response prompting the user to input an identification, which will be compared with data in the user files to ensure that the request is from a person authorised to receive the data. The user files include information relating to users authorised to receive data about particular subjects. Once the user's authority to receive data has been verified, the processor retrieves the information from the relevant subject file and forwarded to the output module, which then sends the data over the communications network 206 to the terminal 202 for display to the user.

## Claims

1. A diagnostic testing system comprising first and second network devices connectable by a communications network:
wherein the first network device (205) comprises:
means for analysing a first part of a blood sample from a subject to generate data representing a lymphocyte count and a neutrophil count;
luminometer means (9) for measuring light emitted from a second part of the blood sample;
means to generate data representing light emitted from the said second part of the blood sample over a period of time; and
means to transmit the generated data over the communications network;
and wherein the second network device (201) comprises: means to receive the generated data;
means to store the generated data in a file associated with the subject;
processing means (104,212) programmed to determine a result indicative of the physical condition, or fitness, of a subject on the basis of the generated light emission data, the generated lymphocyte count and the generated neutrophile count;
means to determine that a fitness result has been generated; and
means to generate a notification in response to that determination.

2. A diagnostic testing system according to claim 1, wherein the data representing light emitted from the said second part of the blood sample over a period of time is recorded in the form of data representing a "luminescence curve".

3. A diagnostic testing system according to claim 1, wherein the second network device includes means to transmit the notification over the communications network to the first network device, or to a third network device.

4. A server (201) for use in the system of claim 1, the server comprising:
means to receive data representing light emitted from the blood sample over a period of time, a lymphocyte count and a neutrophil count of a blood sample from a subject;
means to store the generated data in a file associated with the subject;
processing means (104,212) programmed to determine a result indicative of the physical condition, or fitness, of a subject on the basis of the received light emission data, the lymphocyte count, and the neutrophil count;
means to store the generated fitness result in a file associated with the subject;
means to determine that a fitness result has been generated; and
means to generate a notification in response to that determination.

5. A server according to claim 4, wherein the means to receive data representing light emitted from the said second part of the blood sample over a period of time is adapted to receive the data the form of data representing a "luminescence curve".

6. A server according to claim 4 or claim 5, further including means to transmit the notification over a communications network (206) to a remote termina (202,204) connected to the network.

7. A diagnostic apparatus comprising:
means for receiving a sample of blood from a subject;
means for analysing a first part of the blood sample to generate a lymphocyte count;
means for analysing the first part of the blood sample to generate a neutrophil count; and
luminometer means (9) for measuring light emitted from a second part of the blood sample;
means to generate data representing light emitted from the said second part of the blood sample over a period of time;
processing (104,212) programmed to determine, on the basis of the generated light emission data, the generated lymphocyte count and the generated neutrophil count, a result indicative of the physical condition, or fitness, of a subject; and
means to output the result.

8. A diagnostic apparatus according to claim 7, comprising:
first and second chambers (15,16) for receiving respective parts of the blood sample.

9. A diagnostic apparatus according to claim 8, wherein the first and second chambers are formed in a cartridge (4,40) removably mountable to the apparatus.

10. A diagnostic apparatus according to claim 8 or claim 9, wherein:
the means for analysing the first part of the blood sample comprises means (5) for passing light through the first chamber;
detector means (7) for capturing light which has passed through the first chamber and providing an output signal representative of the captured light; and
processing means to generate a lymphocyte count and a neutrophil count on the basis of the output signal of the detector.

11. A diagnostic apparatus according to claim 10, wherein the detector means captures an image, and the lymphocyte count and a neutrophil count are generated by image processing techniques which identify and count images of the lymphocytes and neutrophils.

12. A diagnostic apparatus according to claim 8 or claim 9, wherein the means for analysing the first part of the blood sample comprises:
a first chamber (15c); a waste collection chamber (15d); a pair of measuring electrodes (55,56); a measuring passage (15b) leading from the first chamber to the waste collection chamber and passing between the measuring electrodes;
means for adding reagents to the first chamber; and
means for passing the first part of the blood sample between through the measuring passage between the two measuring electrodes;
means for measuring inductance between the electrodes; and
processing means for processing the measured data to generate a lymphocyte count and a neutrophil count.

13. A diagnostic apparatus according to claim 7, further including means for adding a chemiluminescent reagent to the second part of the blood sample.

14. A diagnostic apparatus according to claim 13, wherein the means for adding a chemiluminescent reagent comprises a reagent reservoir and dosing means controllable to deliver reagent to a chamber containing a second part of the blood sample.

15. A diagnostic apparatus according to claim 14, further including a timer operable to control the operation of the dosing means.

16. A consumables pack for use with an apparatus according to claim 9, the pack comprising:
a cartridge (40) having a first chamber, a waste collection chamber, a pair of measuring electrodes, and a measuring passage leading from the first chamber to the waste collection chamber and passing between the measuring electrodes, and
at least one syringe (27) pre-filled with a predetermined quantity of a reagent (29) selected from N-formyl-Met-Leu-Phe (fMLP), phorbol 12-myristate 13-acetate (PMA), Pholasin (RTM), luminol and phagoburst (TM).

## Patentansprüche

1. Diagnosetestsystem mit einer ersten und einer zweiten Netzwerkvorrichtung, die durch ein Kommunikationsnetzwerk verbindbar sind,
wobei die erste Netzwerkvorrichtung (205) aufweist:
eine Einrichtung zum Analysieren eines ersten Teils einer Blutprobe von einem Subjekt, um Daten zu erzeugen, die eine Lymphozytenzahl und eine Neutrophilenzahl repräsentieren,
eine Luminometereinrichtung (9) zum Messen von aus einem zweiten Teil der Blutprobe emittiertem Licht,
eine Einrichtung zum Erzeugen von Daten, die aus dem zweiten Teil der Blutprobe über eine Zeitdauer emittiertes Licht repräsentieren, und
eine Einrichtung zum Übertragen der erzeugten Daten über das Kommunikationsnetzwerk, und
wobei die zweite Netzwerkvorrichtung (201) aufweist:
eine Einrichtung zum Empfangen der erzeugten Daten,
eine Einrichtung zum Speichern der erzeugten Daten in einer dem Subjekt zugeordneten Datei,
eine Verarbeitungseinrichtung (104, 212), die dazu programmiert ist, ein Ergebnis zu bestimmen, das den körperlichen Zustand oder die Fitness eines Subjekts auf der Basis der erzeugten Lichtemissionsdaten, der erzeugten Lymphozytenzahl und der erzeugten Neutrophilenzahl angibt,
eine Einrichtung zum Feststellen, dass ein Fitnessergebnis erzeugt wurde, und
eine Einrichtung zum Erzeugen einer Mitteilung in Erwiderung auf diese Feststellung.

2. Diagnosetestsystem nach Anspruch 1, wobei die Daten, die aus dem zweiten Teil der Blutprobe emittiertes Licht über eine Zeitdauer repräsentieren, in Form von Daten aufgezeichnet werden, die eine "Lumineszenzkurve" repräsentieren.

3. Diagnosetestsystem nach Anspruch 1, wobei die zweite Netzwerkvorrichtung eine Einrichtung zum Übertragen der Mitteilung über das Kommunikationsnetzwerk zu der ersten Netzwerkvorrichtung oder zu einer dritten Netzwerkvorrichtung aufweist.

4. Server (201) zur Verwendung in dem System nach Anspruch 1, wobei der Server aufweist:
eine Einrichtung zum Empfangen von Daten, die aus der Blutprobe emittiertes Licht über eine Zeitdauer, eine Lymphozytenzahl und eine Neutrophilenzahl einer Blutprobe aus einem Subjekt repräsentieren,
eine Einrichtung zum Speichern der erzeugten Daten in einer dem Subjekt zugeordneten Datei,
eine Verarbeitungseinrichtung (104, 212), die dazu programmiert ist, ein Ergebnis zu bestimmen, das den körperlichen Zustand oder die Fitness eines Subjekts auf der Basis der empfangenen Lichtemissionsdaten, der Lymphozytenzahl und der Neutrophilenzahl angibt,
eine Einrichtung zum Speichern des erzeugten Fitnessergebnisses in einer dem Subjekt zugeordneten Datei,
eine Einrichtung zum Feststellen, dass ein Fitnessergebnis erzeugt wurde, und
eine Einrichtung zum Erzeugen einer Mitteilung in Erwiderung auf diese Feststellung.

5. Server nach Anspruch 4, wobei die Einrichtung zum Empfangen von Daten, die aus dem zweiten Teil der Blutprobe emittiertes Licht über eine Zeitdauer repräsentieren, dazu ausgelegt ist, die Daten in der Form von Daten zu empfangen, die eine "Lumineszenzkurve" repräsentieren.

6. Server nach Anspruch 4 oder 5, ferner mit einer Einrichtung zum Übertragen der Mitteilung über ein Kommunikationsnetzwerk (206) an eine entfernte Endstelle (202, 204), die an das Netzwerk angeschlossen ist.

7. Diagnosegerät mit
einer Einrichtung zum Empfangen einer Blutprobe aus einem Subjekt,
einer Einrichtung zum Analysieren eines ersten Teils der Blutprobe, um eine Lymphozytenzahl zu erzeugen,
einer Einrichtung zum Analysieren des ersten Teils der Blutprobe, um eine Neutrophilenzahl zu erzeugen, und
einer Luminometereinrichtung (9) zum Messen von aus einem zweiten Teil der Blutprobe emittiertem Licht,
einer Einrichtung zum Erzeugen von Daten, die aus dem zweiten Teil der Blutprobe über eine Zeitdauer emittiertes Licht repräsentieren,
einer Verarbeitungseinrichtung (104, 212), die dazu programmiert ist, auf Basis der erzeugten Lichtemissionsdaten, der erzeugten Lymphozytenzahl und der erzeugten Neutrophilenzahl ein Ergebnis zu bestimmen, das den körperlichen Zustand oder die Fitness eines Subjekts angibt, und
einer Einrichtung zum Ausgeben des Ergebnisses.

8. Diagnosegerät nach Anspruch 7, mit
einer ersten und einer zweiten Kammer (15, 16) zum Empfangen entsprechender Teile der Blutprobe.

9. Diagnosegerät nach Anspruch 8, wobei die erste und die zweite Kammer in einer Kassette (4, 40) gebildet sind, die abnehmbar an dem Gerät befestigt ist.

10. Diagnosegerät nach Anspruch 8 oder 9, wobei
die Einrichtung zum Analysieren des ersten Teils der Blutprobe, eine Einrichtung (5) zum Durchleiten von Licht durch die erste Kammer aufweist,
ferner eine Detektoreinrichtung (7) vorgesehen ist, um Licht, das durch die erste Kammer hindurchgeleitet wurde, zu erfassen und ein für das erfasste Licht repräsentatives Ausgabesignal bereitzustellen, und
ferner eine Verarbeitungsrichtung vorgesehen ist, um eine Lymphozytenzahl und eine Neutrophilenzahl auf Basis des Ausgabesignals des Detektors zu erzeugen.

11. Diagnosegerät nach Anspruch 10, wobei die Detektoreinrichtung ein Bild aufnimmt und die Lymphozytenzahl und eine Neutrophilenzahl durch Bildverarbeitungstechniken erzeugt werden, die Bilder der Lymphozyten und der Neutrophilen identifizieren und zählen.

12. Diagnosegerät nach Anspruch 8 oder 9, wobei die Einrichtung zum Analysieren des ersten Teils der Blutprobe aufweist:
eine erste Kammer (15c),
eine Abfallsammelkammer (15d),
ein Paar Messelektroden (55, 56),
einen Messkanal (15b), der von der ersten Kammer zu der Abfallsammelkammer führt und zwischen den Messelektroden hindurchführt,
eine Einrichtung zum Hinzufügen von Reagenzien zu der ersten Kammer, und
eine Einrichtung zum Hindurchleiten des ersten Teils der Blutprobe durch den Messkanal zwischen den zwei Messelektroden,
eine Einrichtung zum Messen der Induktivität zwischen den Elektroden und
eine Verarbeitungseinrichtung zum Verarbeiten der Messdaten, um eine Lymphozytenzahl und eine Neutrophilenzahl zu erzeugen.

13. Diagnosegerät nach Anspruch 7, ferner mit einer Einrichtung zum Hinzufügen eines Chemilumineszenz-Reagens zu dem zweiten Teil der Blutprobe.

14. Diagnosegerät nach Anspruch 13, wobei die Einrichtung zum Hinzufügen eines Chemilumineszenz-Reagens ein Reagens-Reservoir und eine Dosiereinrichtung aufweist, die zur Ausgabe eines Reagens an eine Kammer steuerbar ist, die einen zweiten Teil der Blutprobe enthält.

15. Diagnosegerät nach Anspruch 14, ferner mit einem Zeitgeber, der zur Steuerung des Betriebs der Dosiereinrichtung betreibbar ist.

16. Verbrauchsmaterialpaket zur Verwendung mit einem Gerät nach Anspruch 9, wobei das Paket aufweist:
eine Kassette (40) mit einer ersten Kammer, einer Abfallsammelkammer, einem Paar Messelektroden und einem Messkanal, der von der ersten Kammer zu der Abfallsammelkammer führt und zwischen den Messelektroden hindurchgeht, und
mindestens eine Spritze (27), die mit einer vorbestimmten Menge eines Reagens (29) vorgefüllt ist, das ausgewählt ist aus N-formyl-Met-Leu-Phe (fMLP), Phorbol-12-myristat-13-acetat (PMA), Pholasin (RTM), Luminol und Phagoburst (TM).

## Revendications

1. Système de test diagnostique comprenant des premier et deuxième dispositifs en réseau connectables par un réseau de communications ;
dans lequel le premier dispositif en réseau (205) comprend :
des moyens pour analyser une première partie d'un échantillon de sang provenant d'un sujet afin de générer des données représentant un compte de lymphocytes et un compte de neutrophiles ;
des moyens formant luminomètre (9) pour mesurer la lumière émise par une deuxième partie de l'échantillon de sang ;
des moyens pour générer des données représentant la lumière émise par ladite deuxième partie de l'échantillon de sang sur une certaine période de temps ; et
des moyens pour transmettre les données générées sur le réseau de communications ;
et dans lequel le deuxième dispositif en réseau (201) comprend :
des moyens pour recevoir les données générées ;
des moyens pour stocker les données générées dans un fichier associé au sujet ;
des moyens de traitement (104, 212) programmés pour déterminer un résultat indicatif de la condition physique, ou de la forme physique, d'un sujet sur la base des données d'émission de lumière générées, du compte de lymphocytes généré et du compte de neutrophiles généré ;
des moyens pour déterminer qu'un résultat de forme physique a été généré ; et
des moyens pour générer une notification en réponse à cette détermination.

2. Système de test diagnostique selon la revendication 1, dans lequel les données représentant la lumière émise par ladite deuxième partie de l'échantillon de sang sur une certaine période de temps sont enregistrées sous la forme de données représentant une "courbe de luminescence".

3. Système de test diagnostique selon la revendication 1, dans lequel le deuxième dispositif en réseau comprend des moyens pour transmettre la notification sur le réseau de communications vers le premier dispositif en réseau, ou vers un troisième dispositif en réseau.

4. Serveur (201) destiné à être utilisé dans le système de la revendication 1, le serveur comprenant :
des moyens pour recevoir des données représentant la lumière émise par l'échantillon de sang sur une certaine période de temps, un compte de lymphocytes et un compte de neutrophiles d'un échantillon de sang provenant d'un sujet ;
des moyens pour stocker les données générées dans un fichier associé au sujet ;
des moyens de traitement (104, 212) programmés pour déterminer un résultat indicatif de la condition physique, ou de la forme physique, d'un sujet sur la base des données d'émission de lumière générées, du compte de lymphocytes généré et du compte de neutrophiles généré ;
des moyens pour stocker le résultat de forme physique généré dans un fichier associé au sujet ;
des moyens pour déterminer qu'un résultat de forme physique a été généré ; et
des moyens pour générer une notification en réponse à cette détermination.

5. Serveur selon la revendication 4, dans lequel les moyens pour recevoir des données représentant la lumière émise par ladite deuxième partie de l'échantillon de sang sur une certaine période de temps sont adaptés pour recevoir les données sous la forme de données représentant une "courbe de luminescence".

6. Serveur selon la revendication 4 ou la revendication 5, comprenant en outre des moyens pour transmettre la notification sur un réseau de communications (206) vers un terminal distant (202, 204) connecté au réseau.

7. Dispositif de diagnostic comprenant :
des moyens pour recevoir un échantillon de sang provenant d'un sujet ;
des moyens pour analyser une première partie de l'échantillon de sang afin de générer un compte de lymphocytes ;
des moyens pour analyser une première partie de l'échantillon de sang afin de générer un compte de neutrophiles ; et
des moyens formant luminomètre (9) pour mesurer la lumière émise par une deuxième partie de l'échantillon de sang ;
des moyens pour générer des données représentant la lumière émise par ladite deuxième partie de l'échantillon de sang sur une certaine période de temps ;
des moyens de traitement (104, 212) programmés pour déterminer, sur la base des données d'émission de lumière générées, du compte de lymphocytes généré et du compte de neutrophiles généré, un résultat indicatif de la condition physique, ou de la forme physique, d'un sujet ; et
des moyens pour délivrer en sortie le résultat.

8. Dispositif de diagnostic selon la revendication 7, comprenant des première et deuxième chambres (15, 16) pour recevoir des parties respectives de l'échantillon de sang.

9. Dispositif de diagnostic selon la revendication 8, dans lequel les première et deuxième chambres sont formées dans une cartouche (4, 40) montée de façon amovible dans le dispositif.

10. Dispositif de diagnostic selon la revendication 8 ou la revendication 9, dans lequel les moyens pour analyser la première partie de l'échantillon de sang comprennent
des moyens (5) pour faire passer de la lumière à travers la première chambre ;
des moyens détecteurs (7) pour capturer la lumière qui a traversé la première chambre et pour délivrer un signal de sortie représentatif de la lumière capturée ; et
des moyens de traitement pour générer un compte de lymphocytes et un compte de neutrophiles sur la base du signal de sortie du détecteur.

11. Dispositif de diagnostic selon la revendication 10, dans lequel les moyens détecteurs capturent une image, et le compte de lymphocytes et un compte de neutrophiles sont générés par des techniques de traitement d'image qui identifient et comptent des images des lymphocytes et des neutrophiles.

12. Dispositif de diagnostic selon la revendication 8 ou la revendication 9, dans lequel les moyens pour analyser la première partie de l'échantillon de sang comprennent :
une première chambre (15c) ;
une chambre de collecte de déchets (15d) ;
une paire d'électrodes de mesure (55, 56) ;
un passage de mesure (15b) allant de la première chambre à la chambre de collecte de déchets et passant entre les électrodes de mesure ;
des moyens pour ajouter des réactifs à la première chambre ; et
des moyens pour faire passer la première partie de l'échantillon de sang à travers le passage de mesure entre les deux électrodes de mesure ;
des moyens pour mesurer l'inductance entre les électrodes ; et
des moyens de traitement pour traiter les données mesurées afin de générer un compte de lymphocytes et un compte de neutrophiles.

13. Dispositif de diagnostic selon la revendication 7, comprenant en outre des moyens pour ajouter un réactif chimioluminescent à la deuxième partie de l'échantillon de sang.

14. Dispositif de diagnostic selon la revendication 13, dans lequel les moyens pour ajouter un réactif chimioluminescent comprennent un réservoir à réactif et des moyens doseurs contrôlables pour délivrer le réactif à une chambre contenant une deuxième partie de l'échantillon de sang.

15. Dispositif de diagnostic selon la revendication 14, comprenant en outre un minuteur opérable pour commander le fonctionnement des moyens doseurs.

16. Pack de consommables destiné à être utilisé avec un dispositif selon la revendication 9, le pack comprenant :
une cartouche (40) comprenant une première chambre, une chambre de collecte de déchets, une paire d'électrodes de mesure, et un passage de mesure allant de la première chambre à la chambre de collecte de déchets et passant entre les électrodes de mesure, et
au moins une seringue (27) remplie à l'avance avec une quantité prédéterminée d'un réactif (29) choisi parmi le N-formyl-Met-Leu-Phe (fMLP), le 12-myristate-13-acétate de phorbol (PMA), le Pholasin (RTM), le luminol et le Phagoburst (TM).
